# FASCICULE DE BREVET EUROPEEN

(11) **EP 2 037 956 B1**
(45) Date de publication et mention de la délivrance du brevet: **12.12.2012**
(21) Numéro de dépôt: 07788861.8
(22) Date de dépôt: 11.06.2007
(51) Int. Cl.: A61K 38/48, A61P 17/00, A61P 29/00

(54) **UTILISATION THERAPEUTIQUE SIMULTANEE, SEPAREE OU ETALEE DANS LE TEMPS D'AU MOINS UNE NEUROTOXINE BOTULIQUE, ET D'AU MOINS UN DERIVE OPIACE**
GLEICHZEITIGE, SEPARATE ODER SEQUENTIELLE VERWENDUNG MINDESTENS EINES BOTULINUS-NEUROTOXINS UND MINDESTENS EINES OPIAT-DERIVATS
SIMULTANEOUS, SEPARATE OR SEQUENTIAL THERAPEUTIC USE OF AT LEAST ONE BOTULINUM NEUROTOXIN AND OF AT LEAST ONE OPIATE DERIVATIVE

(30) Priorité: 16.06.2006 FR 0605368
(43) Date de publication de la demande: 25.03.2009
(73) Titulaire: IPSEN PHARMA, 92100 Boulogne-Billancourt (FR)
(72) Inventeur: AUGUET, Michel, 91120 Palaiseau (FR); FAVRE, Christine, 91530 Saint Maurice Montcouronne (FR); CHABRIER DE LASSAUNIERE, Pierre-Etienne, 75016 Paris (FR)
(74) Mandataire: Retzler, Charlotte
(86) Numéro de dépôt international: PCT/FR2007/000956
(87) Numéro de publication internationale: WO 2007/144493

(56) Documents cités:
- WO-A2-2006/005912
- GB-A- 2 419 526
- US-A1- 2003 138 437
- US-A1- 2005 147 625
- KERN U ET AL: "Long-term treatment of phantom and stump pain with type A botulinum toxin for 1 year. First clinical observations" NERVENARZT, vol. 75, no. 4, avril 2004 (2004-04), pages 336-340, XP002415419 ISSN: 0028-2804
- GORDON D B ET AL: "Pharmacologic management of neuropathic pain" PAIN MANAGEMENT NURSING, W.B. SAUNDERS, vol. 5, décembre 2004 (2004-12), pages 19-33, XP004707534 ISSN: 1524-9042
- HILL A: "Phantom limb pain: a review of the literature on attributes and potential mechanisms.", JOURNAL OF PAIN AND SYMPTOM MANAGEMENT FEB 1999 LNKD- PUBMED:10069153, vol. 17, no. 2, February 1999 (1999-02), pages 125-142, ISSN: 0885-3924
- KIDD B L ET AL: "Mechanisms of inflammatory pain", BRITISH JOURNAL OF ANAESTHESIA, vol. 87, no. 1, July 2001 (2001-07), pages 3-11, ISSN: 0007-0912
- RADHAKRISHNAN ET AL.: "Model of Muscle Pain: Carrageenan Model and Acidic Saline Model", CURRENT PROTOCOLS IN PHARMACOLOGY, vol. Suppl. 25, 2004,

## Description

La présente invention a pour objet un produit comprenant :
- une neurotoxine botulique, et
- un dérivé opiacé ou son sel
pour l'emploi dans le traitement des douleurs inflammatoires, caractérisée en ce que la toxine botulique et le dérivé opiacé ou son sel sont administrés à des doses sub-actives.

La douleur, en particulier la douleur inflammatoire, reste encore aujourd'hui une pathologie difficile à soulager ou à guérir.

Or l'usage des composés actuellement disponibles qui permettent de réduire la douleur de manière satisfaisante est souvent associé à des effets secondaires indésirables (par exemple sédations, accoutumance, hyperalgésie, risque d'ulcères).

Il est donc devenu nécessaire de trouver un moyen pour réduire ces risques d'effets secondaires. Ceci permettrait d'améliorer le traitement de la douleur.

Aussi le problème que se propose de résoudre l'invention est de trouver un nouveau traitement de la douleur inflammatoire.

Les mécanismes d'action de la douleur inflammatoire sont présentés par Kidd et Urban (British Journal of Anaesthesia, 2001, 87(1):3-11).

Kern, et al. 2004 (Nervenartz, 75(4):336-340) décrit l'utilisation de la toxine botulinique de type A pour le traitement de douleurs de membres fantômes chez un patient sous morphine. De multiple théories visant à expliquer l'origine et les mécanismes d'action potentiels de la douleur d'un membre fantôme ont été avancées (voire notamment Anne Hill, 1999, Journal of Pain and Symptom Management, 17(2):125-142). Cependant, aucune d'entres elles ne fait référence à une cause ou composante inflammatoire.

La demande WO2006/005912 est relative au traitement d'une glande, d'un organe ou d'un canal obstrué par un calcul formé naturellement, et mentionne que la toxine botulinique peut être combinée avec un traitment analgésique. Cette publication ne mentionne ni l'utilisation de doses subactives ni le traitment de douleurs inflammatoires.

De manière inattendue, les inventeurs ont mis en évidence que l'association de neurotoxine botulique et de morphine ou d'un analogue ou dérivé de la morphine, présente un effet synergique puissant dans le traitement de la douleur au point de pouvoir réduire considérablement les doses de toxine botulique et de morphine administrées au patient tout en conservant un effet analgésique équivalent. En effet, ces deux principes actifs administrés à des doses subactives (c'est-à-dire à des doses qui ne produisent pas par elles-mêmes d'effet thérapeutique), produisent, lorsqu'ils sont associés, un effet thérapeutique hautement significatif.

Dans ce but la présente invention propose un produit comprenant :
- une neurotoxine botulique, et
- un dérivé opiacé ou son sel
pour l'emploi dans le traitement des douleurs inflammatoires, caractérisée en ce que la toxine botulique et le dérivé opiacé ou son sel sont administrés à des doses sub-actives.

L'invention offre des avantages déterminants, en particulier celui de réduire les doses administrées de morphine. En effet les compositions et produits décrits par l'invention peuvent être utilisés à des doses beaucoup plus faibles comparées aux doses habituellement administrées de morphine actuellement disponible dans le commerce. En d'autres termes, pour obtenir le même effet analgésique, on injecte une quantité inférieure de morphine et de toxine botulique. Pour les mêmes indications thérapeutiques, une réduction comprise entre 10 à 70 %, de préférence entre 25 à 50 %, des doses d'administration a pu être observée (comparaison entre unités de toxines et quantité de morphine (mg/kg) injectées pour obtenir le même effet biologique).

Un autre avantage des compositions décrite par l'invention est qu'ils provoquent peu d'effets secondaires, et notamment beaucoup moins d'effets secondaires que les compositions ou produits connus à ce jour de toxine botulique ou de morphine. En particulier la possibilité d'utiliser de faibles doses de compositions selon l'invention permet avantageusement de réduire les effets secondaires. Parmi les effets secondaires de la toxine botulique qui sont évités, on peut citer ceux liés à l'immunogénicité de la protéine elle-même, ainsi que la dysphagie, la ptose ou la faiblesse musculaire générale, cette liste n'étant pas exhaustive. De même, parmi les effets secondaires principaux de la morphine qui sont évités, on peut citer la constipation, les nausées, les vomissements, la confusion, la somnolence, l'accoutumance et l'hyperalgésie.

Enfin l'invention a pour avantage de pouvoir être mise en oeuvre dans toutes industries, notamment l'industrie pharmaceutique, vétérinaire, cosmétique.

D'autres avantages et caractéristiques de l'invention apparaîtront clairement à la lecture de la description et des exemples donnés à titre purement illustratifs et non limitatifs qui vont suivre.

La présente invention décrite a tout d'abord une composition comprenant :
- une neurotoxine botulique, et
- un dérivé opiacé ou son sel.

Par l'expression neurotoxine botulique, on entend une toxine botulique qui est soit une protéine libre (i.e. libre de toute protéine la complexant), soit un complexe protéique, ledit complexe protéique pouvant comprendre par exemple de l'hémagglutinine (protéine HA) associée à de la toxine botulique, ou soit un fragment protéique.

Par l'expression toxine botulique, on entend une molécule possédant l'activité biologique de la toxine botulique, qui peut être par exemple soit une protéine, soit un polypeptide, soit un peptide, soit une protéine de fusion, soit une protéine tronquée, soit une protéine chimérique, soit une protéine mutée ou une protéine recombinante.

Par l'expression activité biologique de la toxine, on entend au sens de la présente invention soit une paralysie musculaire soit une inhibition de l'exocytose, en particulier de l'exocytose de l'acétylcholine ou d'un autre neurotransmetteur.

Par protéine, polypeptide ou peptide on entend au sens de la présente invention, un polymère d'acides aminés, naturels ou non, lévogyres ou non, dextrogyres ou non.

Par protéine chimérique, on entend au sens de la présente invention une protéine obtenue après association de différents types de molécules, par exemple après association de lipides, de glycolipides, de peptides, de polypeptides, de protéines, de glycoprotéines, de carbohydrates, de polysaccharides, d'acides nucléiques, de polyéthylène glycol, etc.

La toxine botulique, en particulier la toxine botulique de type A1 (Dysport^{®} commercialisé par Ipsen ou Botox^{®} commercialisé par Allergan), est utilisée depuis les années 80 chez l'homme pour le traitement de maladies / désordres divers et variés. Parmi les maladies / désordres pouvant être traités par la toxine botulique, on peut citer entre autres des désordres musculaires (par exemple le blépharospasme, la spasticité de l'adulte ou de l'enfant ou encore le torticolis), la migraine, la douleur d'origine musculaire, la douleur neuropathique, le diabète, l'hyperhydrose (ou transpiration excessive), l'hypersalivation ou même les rides.

Les applications des toxines botuliques connues à ce jour ont trait à son administration classique, intra-musculaire telle que décrite dans les traitements mentionnés. L'injection dans les muscles provoque leur paralysie transitoire, c'est à dire bloque les contractions musculaires sur une certaine période de temps.

La neurotoxine botulique, pure ou quasiment pure, peut être obtenue à partir d'un complexe protéique comprenant de la toxine botulique par exemple selon la méthode décrite dans Current topics in Microbiology and Immunology (1995), 195, p. 151-154. Une neurotoxine botulique, pure ou quasiment pure, peut être obtenue par exemple, par purification d'un milieu de fermentation ou bouillon de culture contenant une souche de *Clostridium Botulinum,* et enrichi par exemple en viande ou en nourriture protéinée.

La composition décrite par l'invention comprend :
- au moins une neurotoxine botulique, et
- au moins un dérivé opiacé ou son sel.

De préférence la composition selon l'invention comprend au moins une neurotoxine botulique de type A, A1, A2, B, C, C1, D, E, F ou G.

La neurotoxine botulique de type A1 correspond en fait à la toxine botulique classique qui est communément appelée toxine botulique de type A, sans distinction du sous-type. La neurotoxine botulique de type A1 est commercialisée sous le nom de DYSPORT^{®} ou RELOXIN^{®} ou BOTOX^{®}.

Selon l'invention, la neurotoxine botulique de type A1 peut correspondre soit à un complexe de toxine botulique A1 et d'hémagglutinine, soit à la toxine botulique de type A1 libre de toutes protéines complexantes.

La toxine botulique de type A2 a d'abord été isolée à partir de cas d'enfants atteints de botulisme vers 1990 (Sakaguchi et al., Int. J. Food Microbiol. (1990), 11, 231-242). Cette toxine est immunologiquement et biochimiquement différente de la toxine botulique de type A1.

La toxine botulique de type A2 peut être isolée à partir des souches suivantes : Kyoto-F, Chiba-H, Y-8036, 7103-H, 7105-H, KZ1828, NCTC2012 ou NCTC9837 (Cordoba et al., System. Appl. Microbiol. (1995), 18, 13-22; Franciosa et al., abstract presented at 40th Interagency Botulism Research Coordinating Committee (IBRCC) Meeting, November 2003).

De préférence la composition selon l'invention comprend la toxine botulique de type A1.

Selon une variante la composition selon l'invention comprend la toxine botulique de type A2 isolée à partir de la souche *Clostridium botulinum* référencée et accessible sous le numéro NCTC9837, auprès de la National Collection of Type Cultures - Central Public Health Laboratory - London - UK. La souche NCTC9837 est parfois appelée souche Mauritius 1955.

La toxine botulique de type A2 diffère de la toxine A1 par entre autre, sa séquence en acides aminés, son poids moléculaire, ses caractéristiques immunologiques et génétiques (Kubota et al., Biochem. Biophys. Res. Commun. (1996), 224 (3), 843-848).

La composition selon l'invention comprend au moins un dérivé opiacé ou son sel.

Par l'expression « dérivé opiacé ou son sel », on entend au sens de la présente invention, les substances communément appelées « opiacées », et en particulier les analogues ou dérivés de la morphine. En effet la morphine, aujourd'hui bien connue pour ses effets anti-douleur, a quant à elle été isolée au tout début du XIX^{e} siècle par un pharmacien allemand, Friedrich Sertürner, à partir de l'opium dont elle est le constituant principal.

Selon une variante, la composition selon l'invention comprend au moins un dérivé opiacé ou son sel choisi parmi les analogues ou dérivés de la morphine, le fentanyl, l'alfentanil, la codéine, la dihydrocodéine, l'hydrocodone, l'oxycodone, l'hydromorphone, la péthidine, le rémifentanyl, le sufentanil, le dextropropoxyphène, le tramadol, la buprénorphine, la nalbuphine, le sulfate de morphine, le chlorhydrate d'hydromorphone ou le sulfate de morphine enrobé.

Par sel, on entend un sel pharmaceutiquement acceptable et notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt sélection for basic drugs", Int. J. Pharm. (1986), 33, 201-217.

La présente composition décrite par l'invention peut en outre comprendre au moins un polysaccharide ou un mélange de plusieurs polysaccharides.

Par polysaccharide, on entend au sens de la présente invention, un polymère comprenant au moins 2 monomères, les monomères étant des saccharides. Cette définition inclut les disaccharides.

Dans le cadre de l'invention, les polysaccharides peuvent être ioniques et/ou non ioniques.

De préférence, la composition comprend au moins un polysaccharide comprenant majoritairement des unités de glucose. Le terme majoritairement signifiant, que le glucose est majoritaire en nombre d'unités de monomère.

Comme exemple de polysaccharides convenant selon l'invention, on peut citer l'amidon, les dérivés d'amidon, l'hydroxyethyle amidon en particulier le 2-hydroxy-ethyl amidon.

Les polysaccharides convenant selon la présente invention peuvent être substitués, en particulier peuvent être substitués par des radicaux alkyle, alcoxy, ou encore par des radicaux alkyle eux-mêmes substitués par des fonctions alcools.

Selon une variante de l'invention, la quantité de polysaccharide comprise dans la composition selon l'invention est d'au moins 1 µg de polysaccharide pour 1 unité de toxine botulique. Selon le choix du polysaccharide, il est possible d'utiliser au moins 0,5 µg de polysaccharide pour 1 unité de toxine botulique.

La présente composition décrite par l'invention peut en outre comprendre au moins un tensio-actif ou un mélange de plusieurs tensio-actifs.

Par agent tensio-actif, on entend au sens de l'invention un agent émulsifiant ou un agent solubilisant.

Dans le cadre de l'invention les tensio-actifs mis en oeuvre peuvent être choisis parmi les tensio-actifs cationiques, anioniques ou non-ioniques.

De préférence la composition selon l'invention comprend au moins un tensio-actif choisi parmi les tensio-actifs non-ioniques du groupe des polysorbates.

Parmi le groupe des polysorbates, on peut citer le polysorbate 20, le polysorbate 21, le polysorbate 40, le polysorbate 60, le polysorbate 61, le polysorbate 65, le polysorbate 80, le polysorbate 81, le polysorbate 85, le polysorbate 120, le polysorbate 80 acetate.

Le tensio-actif préféré selon une variante de la composition selon l'invention est le polysorbate 80.

La composition décrite par l'invention peut être sous forme solide, par exemple des poudres, des lyophilisats, des granules, des comprimés ou des liposomes. La composition selon l'invention sous forme solide peut être conservée par exemple à des températures inférieures à 4°C, ou inférieures à 0°C sans que son activité biologique soit altérée.

La composition peut se présenter sous forme d'une dispersion aqueuse, de particules de neurotoxine botulique dans un réseau gélifié.

La composition peut aussi se présenter sous forme liquide, par exemple, des solutions, des émulsions ou des suspensions.

L'administration de la composition se fera de préférence par injection comme par exemple par injection intramusculaire ou sous-cutanée.

Dans le cas des injections, la composition pourra être associée à un agent facilitant l'injection encore appelé véhicule d'injection ou vecteur d'injection.

Selon un mode de réalisation des compositions selon l'invention, le ratio des fractions d'unité de neurotoxine botulique sur la quantité de dérivé opiacé ou son sel [unités BT /mg dérivé opiacé] peut être compris entre 0,1 / 1000 et 1000 / 0,1, de préférence entre 80 / 200 et 200 / 80, encore plus préférentiellement entre 10 / 333 et 333 / 10, avantageusement entre 0,3 / 10 et 10 / 0,3.

La composition décrite ci-dessus peut être utilisée pour l'obtention d'un médicament destiné à traiter ou prévenir la douleur, en particulier les douleurs liées à un cancer, les douleurs liées à des maladies chroniques autres qu'un cancer, les douleurs neuropathiques, les douleurs liées à des radiculopathies, à des neuropathies diabétiques ou liées au SIDA ou consécutives au SIDA ou aux agents anti-cancéreux, les douleurs inflammatoires, l'adiposis dolorosa, les douleurs liées aux brûlures, la migraine, les douleurs pré- et post-opératoires, les douleurs inflammatoires chroniques, la sciatique, les neuralgies post herpétiques, la fibromyalgie, l'algoneurodystrophie ou syndrome douloureux régional complexe et les douleurs centrales consécutives aux accidents cérébraux vasculaires, aux lésions thalamiques ou à la sclérose en plaques (multiple), ou les douleurs physiques : traumatismes, ou les douleurs liées à une intoxication.

La composition décrite ci-dessus peut être utilisée pour l'obtention d'un médicament destiné à traiter ou prévenir les défauts cosmétiques, les désordres musculaires, les désordres neuromusculaires, les désordres neurologiques, les désordres orthopédiques, les désordres ophtalmologiques, les désordres psychologiques, les pathologies articulaires, les désordres endocriniens ou les désordres urologiques.

La composition décrite ci-dessus peut être utilisée pour l'obtention d'un médicament destiné à traiter ou prévenir le torticolis, le torticolis spasmodique, les troubles locaux de la spasticité des membres supérieurs et/ou inférieurs, la douleur, la douleur musculaire, la douleur due aux spasmes musculaires, la douleur myofaciale, les douleurs post-opératoires, les spasmes musculaires, le spasme hémifacial, le blépharospame, le strabisme, l'asymétrie faciale, la dystonie musculaire, la paralysie cérébrale, les céphalées, la migraine, la fibromyalgie, la myalgie, les états dépressifs, l'hyperhydrose, la bromhydrose, la coxarthrose, l'arthrose de la hanche, l'épicondylite du coude, l'arthrite, l'arthrite rhumatoïde, les dyskinésies, l'achalasie, les dysfonctionnements des sphincters de Oddi, la pancréatite, la goutte, les fissures anales, la constipation, l'anismus, les spasmes de la valve pylorique, la vessie spastique, les spasmes de la vessie, l'incontinence urinaire, la rétention d'urine, l'hyperplasie prostatique, l'endométriose, le psoriasis, la rhinite, la rhinite allergique, l'obésité, l'hyperlacrimation, les fractures osseuses, les déchirures des tendons ou la pathologie de la coiffe des muscles rotateurs de l'épaule.

La composition décrite ci-dessus peut également être utilisée pour l'obtention d'un produit cosmétique.

La composition décrite ci-dessus peut également être utilisée pour l'obtention d'un médicament destiné à traiter ou prévenir les lignes de froncement du visage, les rides du visage, les rides de la peau, les rides du contour de l'oeil, les rides de la glabelle, les sillons intersourcilliers, la calvitie, l'acné, la transpiration excessive ou la perte des cheveux.

La composition peut être utilisée pour obtenir un médicament destiné à traiter ou prévenir la douleur.

Par « douleur » au sens de la présente invention, il faut entendre « toute expérience désagréable émotionnelle et sensorielle associée un dommage tissulaire présent ou potentiel ou décrite par le patient en de tels termes ».

Parmi les douleurs pouvant être traitées par une composition ou un produit décrit par l'invention, on pourra citer notamment :
❖ les douleurs liées à un cancer (particulièrement préférées dans la mesure où la neurotoxine botulique est également un agent anti-cancéreux) ;
❖ les douleurs liées à des maladies chroniques autres qu'un cancer telles que les douleurs liées à des maladies virales ou rétrovirales (par exemple les douleurs liées au Syndrome Immuno-Déficitaire Acquis (SIDA) ou les douleurs liées au zona) ou les douleurs liées à des neuropathies diabétiques ;
❖ les douleurs neuropathiques telles que la névralgie du trijumeau, les névralgies glosso-pharyngiennes, les douleurs liées à des radiculopathies et les douleurs liées à des neuropathies secondaires à des infiltrations métastasiques ;
❖ l'adiposis dolorosa ;
❖ les douleurs liées aux brûlures ;
❖ la douleur due à une migraine ;
❖ les douleurs pré- et post-opératoires ;
❖ les douleurs chroniques, la fibromyalgie, l'algoneurodystrophie ou syndrome douloureux régional complexe (« *Complex Regional Pain Syndrome* » en anglais) ;
❖ les douleurs centrales consécutives aux accidents cérébraux vasculaires, aux lésions thalamiques ou à la sclérose en plaques.

La dose de la composition décrite par la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

La quantité thérapeutiquement efficace qu'il convient d'injecter varie également selon le nombre de muscles à traiter, ainsi que selon la masse de ces muscles.

De préférence les doses injectées de neurotoxines botuliques, comprises dans la composition selon l'invention, seront comprises entre 0,1 et 1000 Unités de toxine botulique, plus préférentiellement de 1 à 500 Unités de toxine botulique, encore plus préférentiellement de 5 à 100 Unités de toxine botulique, et ceci quel que soit le type de toxine botulique ou quelle que soit sa provenance.

De préférence les doses injectées de morphine seront comprises entre 0,001 et 0,3 mg par kg / jour.

Un produit décrit par la présente invention comprenant au moins une neurotoxine botulique et au moins un dérivé opiacé ou son sel est également décrit comme produit de combinaison pour une utilisation thérapeutique simultanée, séparée ou étalée dans le temps dans le traitement ou la prévention de la douleur, des défauts cosmétiques, des désordres musculaires, des désordres neuromusculaires, des désordres neurologiques, des désordres orthopédiques, des désordres ophtalmologiques, des désordres psychologiques, des pathologies articulaires, des désordres endocriniens ou des désordres urologiques.

Par utilisation thérapeutique simultanée, au sens de la présente invention, on entend dans la présente demande une administration d'au moins 2 principes actifs par la même voie et au même moment ou sensiblement au même moment.

Par utilisation séparée, au sens de la présente invention, on entend notamment une administration d'au moins 2 principes actifs au même moment ou sensiblement au même moment par des voies différentes.

Par utilisation thérapeutique étalée dans le temps, on entend une administration d'au moins 2 principes actifs à des moments différents, la voie d'aministration étant identique ou différente. Plus particulièrement, on entend un mode d'administration selon lequel l'ensemble de l'administration de l'un des principes actifs est effectué avant que l'administration de l'autre ou des autres ne commence. On peut ainsi administrer l'un des principes actifs pendant plusieurs mois avant d'administrer l'autre ou les autres principes actifs. Il n'y a pas de traitement simultané dans ce cas. On peut aussi envisager une administration alternée de chaque principe actif pendant plusieurs semaines.

Le produit décrit par la présente invention peut être utilisé dans le traitement ou la prévention de la douleur, en particulier les douleurs liées à un cancer, les douleurs liées à des maladies chroniques autres qu'un cancer, les douleurs neuropathiques, les douleurs liées à des radiculopathies, à des neuropathies diabétiques ou liées au SIDA ou consécutives au SIDA ou aux agents anti-cancéreux, les douleurs inflammatoires, l'adiposis dolorosa, les douleurs liées aux brûlures, la migraine, les douleurs pré- et post-opératoires, les douleurs inflammatoires chroniques, la sciatique, les neuralgies post herpétiques, la fibromyalgie, l'algoneurodystrophie ou syndrome douloureux régional complexe et les douleurs centrales consécutives aux accidents cérébraux vasculaires, aux lésions thalamiques ou à la sclérose en plaques (multiple), ou les douleurs physiques : traumatismes, amputations, ou les douleurs liées à une intoxication.

Le produit décrit par la présente invention peut être utilisé dans le traitement ou la prévention des troubles choisis parmi le torticolis, le torticolis spasmodique, les troubles locaux de la spasticité des membres supérieurs et/ou inférieurs, la douleur, la douleur musculaire, la douleur due aux spasmes musculaires, la douleur myofaciale, les douleurs post-opératoires, les spasmes musculaires, le spasme hémifacial, le blépharospame, le strabisme, l'asymétrie faciale, la dystonie musculaire, la paralysie cérébrale, les céphalées, la migraine, la fibromyalgie, la myalgie, les états dépressifs, l'hyperhydrose, la bromhydrose, la coxarthrose, l'arthrose de la hanche, l'épicondylite du coude, l'arthrite, l'arthrite rhumatoïde, les dyskinésies, l'achalasie, les dysfonctionnements des sphincters de Oddi, la pancréatite, la goutte, les fissures anales, la constipation, l'anismus, les spasmes de la valve pylorique, la vessie spastique, les spasmes de la vessie, l'incontinence urinaire, la rétention d'urine, l'hyperplasie prostatique, l'endométriose, le psoriasis, la rhinite, la rhinite allergique, l'obésité, l'hyperlacrimation, les fractures osseuses, les déchirures des tendons ou la pathologie de la coiffe des muscles rotateurs de l'épaule.

Le produit décrit par la présente invention peut être utilisé dans le traitement ou la prévention des troubles des lignes de froncement du visage, des rides du visage, des rides de la peau, des rides du contour de l'oeil, des rides de la glabelle, des sillons intersourcilliers, de la calvitie, de l'acné, de la transpiration excessive ou de la perte des cheveux.

Le produit décrit par la présente invention peut également être utilisé dans le traitement ou la prévention de la douleur, telle que définie ci-dessus.

La neurotoxine botulique mise en oeuvre dans le produit peut comprendre au moins une neurotoxine botulique de type A, A1, A2, B, C, C1, D, E, F ou G.

Le dérivé opiacé ou son sel mis en oeuvre dans le produit est choisi parmi les analogues ou dérivés de la morphine, le fentanyl, l'alfentanil, la codéine, la dihydrocodéine, l'hydrocodone, l'oxycodone, l'hydromorphone, la péthidine, le rémifentanyl, le sufentanil, le dextropropoxyphène, le tramadol, la buprénorphine, la nalbuphine, le sulfate de morphine, le chlorhydrate d'hydromorphone ou le sulfate de morphine enrobé.

Selon une variante, le produit comprend au moins une neurotoxine botulique de type A, A1, A2, B, C, C1, D, E, F ou G, et au moins un dérivé opiacé ou son sel choisi parmi les analogues ou dérivés de la morphine, le fentanyl, l'alfentanil, la codéine, la dihydrocodéine, l'hydrocodone, l'oxycodone, l'hydromorphone, la péthidine, le rémifentanyl, le sufentanil, le dextropropoxyphène, le tramadol, la buprénorphine, la nalbuphine, le sulfate de morphine, le chlorhydrate d'hydromorphone ou le sulfate de morphine enrobé.

D'une manière générale, ce qui a été écrit plus haut concernant la neurotoxine botulique comprise dans la composition selon l'invention est valable pour la neurotoxine botulique comprise dans le produit.

Ce qui a été écrit plus haut concernant le dérivé opiacé ou son sel compris dans la composition selon l'invention est valable pour le dérivé opiacé ou son sel compris dans le produit.

Le produit, et en particulier la neurotoxine botulique, peut en outre comprendre au moins un polysaccharide ou un mélange de plusieurs polysaccharides tel que décrit plus haut, au moins un tensio-actif ou un mélange de plusieurs tensio-actifs tel que décrit plus haut.

Le produit peut se présenter entre autre sous forme d'un solide, d'un liquide ou d'une dispersion aqueuse, tel que décrit plus haut.

La voie d'administration du produit peut être par injection comme par exemple par injection intramusculaire ou sous-cutanée pour la neurotoxine botulique.

La voie d'administration du produit peut être par voie topique, orale, parentérale, par injection intramusculaire, intraveineuse, sous-cutanée etc. pour le dérivé opiacé ou son sel.

La voie d'administration du produit est de préférence par injection intramusculaire pour la neurotoxine botulique et de préférence par voie orale pour le dérivé opiacé ou son sel.

Dans le cas des injections, le produit pourra être associé à un agent facilitant l'injection encore appelé véhicule d'injection ou vecteur d'injection.

Selon un mode de réalisation des compositions selon l'invention, le ratio des fractions d'unité de neurotoxine botulique sur la quantité de dérivé opiacé ou son sel [unités BT /mg dérivé opiacé] peut être compris entre 0,1 / 1000 et 1000 / 0,1, de préférence entre 80 / 200 et 200 / 80, encore plus préférentiellement entre 10 / 333 et 333 / 10, avantageusement entre 0,3 / 10 et 10 / 0,3.
La figure 1 présente l'effet de la toxine botulique de type A suite à l'injection par voie sous-plantaire dans le modèle d'hyperalgésie inflammatoire induite par la carragénine.
La figure 2 présente l'effet suite à l'injection par voie intra-péritonéale de morphine dans le modèle d'hyperalgésie inflammatoire induite par la carragénine.
La figure 3 présente l'effet suite aux injections étalées dans le temps de toxine botulique de type A par voie sous-plantaire et de morphine par voie intra-péritonéale dans le modèle d'hyperalgésie inflammatoire induite par la carragénine.
La figure 4 présente le protocole d'injection.

Les exemples suivants illustrent l'invention sans en limiter la portée.

### EXEMPLES

La quantification des neurotoxines botuliques utilisées selon l'invention a été réalisée par la mesure d'une dose létale DL₅₀. Par DL₅₀ on entend au sens de la présente invention la dose létale ou encore dose semi létale d'une substance donnée. Il s'agit de la dose (ou quantité) qui conduit à la mort de 50% des animaux testés d'un groupe. Une unité de toxine (U) correspond à la DL₅₀ chez la souris par voie intra péritonéale.

### Etude pharmacologique des produits de l'invention

L'activité des compositions de l'invention a été évaluée in vivo sur un modèle d'hyperalgésie induite par la carragénine sur la patte de rat.

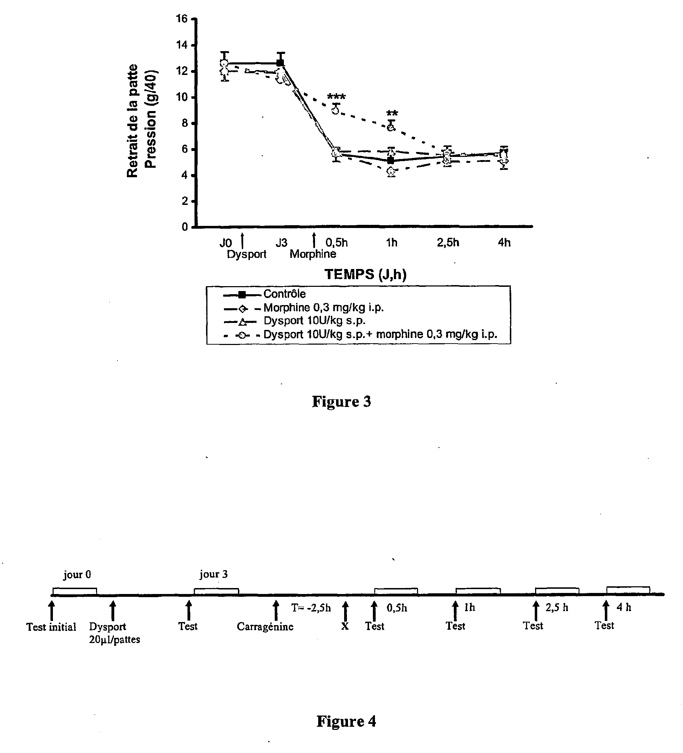

Des rats mâles Sprague Dawley (Charles River) de 190 à 210 g le jour de l'expérience sont laissés en stabulation 5 à 8 jours dans les conditions d'animalerie et sont mis à jeun sur grilles 18 h avant et pendant l'expérience. Les groupes sont constitués d'au moins 8 animaux. La toxine botulique (Dysport) ou son véhicule est administré, 3 jours avant l'expérience d'hyperalgésie à la carragénine, par voie sous-plantaire (s.p., 20µl/patte) dans les deux pattes arrières du rat. La morphine (X sur la figure 4) ou son véhicule est administré, le jour de l'expérience, par voie intrapéritonéale (i.p., 2 ml/kg), 2 h 30 après l'injection de carragénine. La carragénine 2% a été injectée par voie sous-plantaire dans la patte arrière droite des rats. Le seuil de la douleur (ou seuil nociceptif) a été évalué en mesurant le retrait de la patte du rat suscité par un stimulus mécanique dont la pression est croissante (pression initiale de 210 g/mm²) appliqué à l'aide d'un analgésie-mètre (test de Randall-Selitto). Les mesures ont été faites avant l'injection de toxine botulique (test initial à J0) et juste avant l'injection de carragénine (J3 ou t = - 2 h 30) et 30 min (ou 3h après la carragénine), 1 h, 2h30 et 4h après l'injection de la morphine ou de son véhicule (effectuée à t = 0).

Dans l'expérience avec la toxine seule, le protocole expérimental est identique à celui cité ci-dessus sans l'injection de morphine ou de son véhicule, l'expérience d'hyperalgésie à la carragénine s'effectue à J3 et la première mesure d'activité au même temps de 3h après la carragénine (soit 2h30 +30 min).

Dans l'expérience avec la morphine seule, le protocole expérimental est identique à celui cité ci-dessus sans l'injection de toxine botulique ou de son véhicule à J0, les mesures des effets analgésiques s'effectuent juste avant l'injection de carragénine (t = - 2 h 30) et 30 min (ou 3h après la carragénine), et 2h30 après l'injection de la morphine ou de son véhicule (effectuée à t = 0).

L'efficacité des produits est évaluée par leur capacité à réduire de manière significative l'hyperalgésie induite par la carragénine. Cette efficacité sera statistiquement déterminée par un Student's test et Satterthwaite's test (deux voies).

Le protocole expérimental se situe à la figure 4.

### Exemple 1 : Effet de la toxine botulique (Dysport®) sur l'hyperalgésie induite par la carragénine :

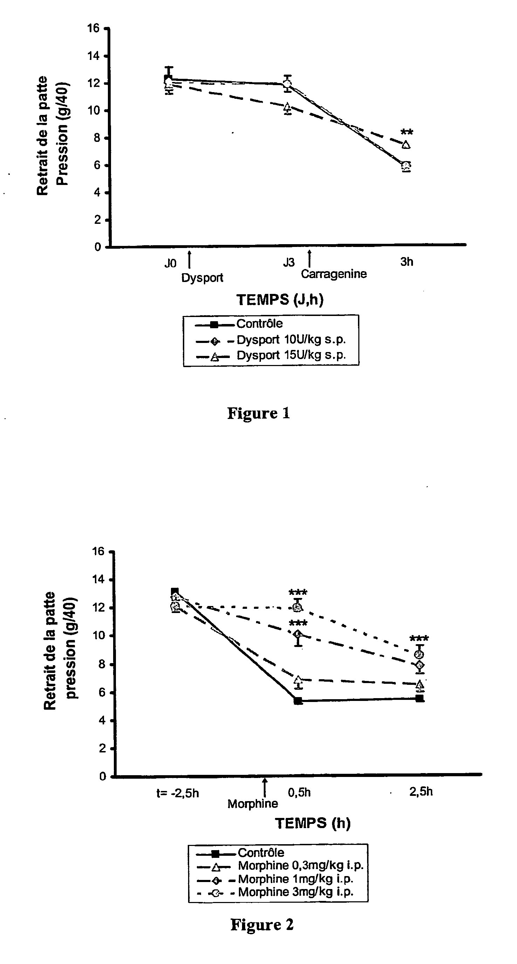

Les résultats obtenus en utilisant le Dysport^{®} dans le modèle d'hyperalgésie induite par la carragénine sur la patte de rat décrit ci-dessus sont reportés dans la Figure 1.

L'effet analgésique du Dysport^{®} est démontré dans le test d'hyperalgésie induite par la carragénine. A la dose de 15 U/kg (s.p.) et au 3^{ème} jour, le seuil de douleur suite à un stimulus mécanique appliqué sur les pattes des rats, est augmenté de 46% alors qu'à la dose de 10 U/kg aucune protection statistiquement significative de l'hyperalgésie à la carragénine n'est observée.

### Exemple 2: Effet de la morphine sur l'hyperalgésie induite par la carragénine :

Les résultats obtenus en utilisant de la morphine dans le modèle d'hyperalgésie induite par la carragénine sur la patte de rat décrit ci-dessus sont reportés dans la Figure 2.

L'effet analgésique de la morphine est démontré dans le test d'hyperalgésie induite par la carragénine. A partir de la dose de 1 mg/kg (i.p.) le seuil de douleur suite à un stimulus mécanique appliqué sur les pattes des rats, est augmenté de 65% et jusqu'à 97% pour la dose de 3 mg/kg alors que la dose de 0.3mg/kg n'induit pas de protection statistiquement significative de l'hyperalgésie à la carragénine.

### Exemple 3: Effet de l'association de Dysport^{®} et de morphine sur l'hyperalgésie induite par la carragénine :

Les résultats obtenus en utilisant une association de Dysport^{®} et de morphine dans le modèle d'hyperalgésie induite par la carragénine sur la patte de rat qui est décrit ci-dessus, sont reportés dans la figure 3. Le protocole d'administration est indiqué à la figure 4, le terme « X » correspond à l'injection de la morphine ou de son véhicule.

Un effet analgésique est observé avec la combinaison de doses non actives de Dysport (10U/kg s.p.) et de morphine (0.3mg/kg i.p.) (autrement dit, on observe un effet synergique). En comparant avec les résultats des exemples 1 et 2, on constate donc que l'administration de Dysport^{®} et de morphine a un effet analgésique synergique lorsqu'ils sont associés. En effet, l'utilisation de Dysport^{®} permet de réduire d'au moins un facteur 2 les doses de morphine nécessaires pour obtenir un effet équivalent. De la même façon, l'utilisation de morphine permet de réduire d'au moins un facteur supérieur à 1,5 les doses de Dysport nécessaires pour obtenir un effet équivalent.

## Revendications

1. Produit comprenant :
- une neurotoxine botulique, et
- un dérivé opiacé ou son sel
pour l'emploi dans le traitement des douleurs inflammatoires, **caractérisée en ce que** la toxine botulique et le dérivé opiacé ou son sel sont administrés à des doses sub-actives.

2. Produit selon la revendication 1 pour l'emploi dans le traitement des douleurs inflammatoires, **caractérisée en ce qu'**il comprend au moins une neurotoxine botulique de type A, A1, A2, B, C, C1, D, E, F ou G.

3. Produit selon l'une des revendications précédentes pour l'emploi dans le traitement des douleurs inflammatoires, **caractérisée en ce qu'**il comprend au moins un dérivé opiacé ou son sel choisi parmi les analogues ou dérivés de la morphine, le fentanyl, l'alfentanil, la codéine, la dihydrocodéine, l'hydrocodone, l'oxycodone, l'hydromorphone, la péthidine, le rémifentanyl, le sufentanil, le dextropropoxyphène, le tramadol, la buprénorphine, la nalbuphine, le sulfate de morphine, le chlorhydrate d'hydromorphone ou le sulfate de morphine enrobé.

4. Produit selon l'une des revendications précédentes pour l'emploi dans le traitement des douleurs inflammatoires, **caractérisée en ce qu'**il comprend au moins un polysaccharide ou un mélange de plusieurs polysaccharides.

5. Produit selon l'une des revendications précédentes pour l'emploi dans le traitement des douleurs inflammatoires, **caractérisée en ce qu'**il comprend au moins un polysaccharide qui est le 2-hydroxy-ethyl amidon.

6. Produit selon l'une des revendications précédentes pour l'emploi dans le traitement des douleurs inflammatoires, **caractérisée en ce qu'**il comprend au moins un tensio-actif ou un mélange de plusieurs tensio-actifs, choisi parmi les tensio-actifs cationiques, anioniques ou non-ioniques.

7. Produit selon l'une des revendications précédentes pour l'emploi dans le traitement des douleurs inflammatoires, **caractérisée en ce qu'**il comprend au moins un tensio-actif choisi parmi les tensio-actifs non-ioniques du groupe des polysorbates.

8. Produit selon l'une des revendications précédentes pour l'emploi dans le traitement des douleurs inflammatoires, **caractérisée en ce que** la toxine botulique est un complexe protéique.

9. Produit selon l'une des revendications précédentes pour l'emploi dans le traitement des douleurs inflammatoires, **caractérisée en ce que** la toxine botulique est une protéine libre de toute protéine la complexant.

10. Produit selon l'une des revendications précédentes pour l'emploi dans le traitement des douleurs inflammatoires, **caractérisée en ce que** la toxine botulique est une protéine recombinante.

11. Produit selon l'une des revendications précédentes pour l'emploi dans le traitement des douleurs inflammatoires, **caractérisée en ce que** la toxine botulique est administrée par injection intramusculaire ou sous-cutanée.

12. Produit selon l'une des revendications précédentes pour l'emploi dans le traitement des douleurs inflammatoires, **caractérisée en ce que** le dérivé opiacé ou son sel est administré par une voie choisie parmi la voie topique, orale, parentérale, intramusculaire, intraveineuse ou sous-cutanée.

13. Produit selon l'une des revendications précédentes pour l'emploi dans le traitement des douleurs inflammatoires, **caractérisée en ce qu'**il se présente sous forme liquide.

## Claims

1. Product comprising:
- a botulinum neurotoxin, and
- an opiate derivative or its salt.
for use in the treatment of inflammatory pain, **characterized in that** the botulinum toxin and the opiate derivative or its salt are administered in subactive doses.

2. Product according to claim 1 for use in the treatment of inflammatory pain, **characterized in that** it comprises at least one botulinum neurotoxin of type A, A1, A2, B, C, C1, D, E, F or G.

3. Product according to one of the previous claims for use in the treatment of inflammatory pain, **characterized in that** it comprises at least one opiate derivative or its salt chosen from the morphine analogues or derivatives, fentanyl, alfentanil, codeine, dihydrocodeine, hydrocodone, oxycodone, hydromorphone, pethidine, remifentanyl, sufentanil, dextropropoxyphene, tramadol, buprenorphine, nalbuphine, morphine sulphate, hydromorphone hydrochloride or coated morphine sulphate.

4. Product according to one of the previous claims for use in the treatment of inflammatory pain, **characterized in that** it comprises at least one polysaccharide or a mixture of several polysaccharides.

5. Product according to one of the previous claims for use in the treatment of inflammatory pain, **characterized in that** it comprises at least one polysaccharide which is 2-hydroxy-ethyl starch.

6. Product according to one of the previous claims for use in the treatment of inflammatory pain, **characterized in that** it comprises at least one surfactant or a mixture of several surfactants, chosen from the cationic, anionic or non-ionic surfactants.

7. Product according to one of the previous claims for use in the treatment of inflammatory pain, **characterized in that** it comprises at least one surfactant chosen from the non-ionic surfactants of the polysorbates group.

8. Product according to one of the previous claims for use in the treatment of inflammatory pain, **characterized in that** the botulinum toxin is a protein complex.

9. Product according to one of the previous claims for use in the treatment of inflammatory pain, **characterized in that** the botulinum toxin is a protein free of any protein complexing it.

10. Product according to one of the previous claims for use in the treatment of inflammatory pain, **characterized in that** the botulinum toxin is a recombinant protein.

11. Product according to one of the previous claims for use in the treatment of inflammatory pain, **characterized in that** the botulinum toxin is administered by intramuscular or sub-cutaneous injection.

12. Product according to one of the previous claims for use in the treatment of inflammatory pain, **characterized in that** the opiate derivative or its salt is administered by a route chosen from the topical, oral, parenteral, intramuscular, intravenous or sub-cutaneous route.

13. Product according to one of the previous claims for use in the treatment of inflammatory pain, **characterized in that** it is presented in liquid form.

## Patentansprüche

1. Produkt, umfassend:
- ein Botulinum-Neurotoxin und
- ein Opiatderivat oder sein Salz,
zur Verwendung bei der Behandlung von Entzündungsschmerzen, **dadurch gekennzeichnet, dass** das Botulinum-Toxin und das Opiatderivat oder sein Salz in subwirksamen Dosen verabreicht werden.

2. Produkt nach Anspruch 1 zur Verwendung bei der Behandlung von Entzündungsschmerzen, **dadurch gekennzeichnet, dass** es wenigstens ein Botulinum-Neurotoxin des Typs A, A1, A2, B, C, C1, D, E, F oder G umfasst.

3. Produkt nach einem der vorangehenden Ansprüche zur Verwendung bei der Behandlung von Entzündungsschmerzen, **dadurch gekennzeichnet, dass** es wenigstens ein Opiatderivat oder sein Salz, ausgewählt aus Analogen oder Derivaten von Morphin, Fentanyl, Alfentanil, Codein, Dihydrocodein, Hydrocodon, Oxycodon, Hydromorphon, Pethidin, Remifentanyl, Sufentanil, Dextropropoxyphen, Tramadol, Buprenorphin, Nalbuphin, Morphinsulfat, Hydromorphon-Hydrochlorid oder Morphinsulfat, ummantelt, umfasst.

4. Produkt nach einem der vorangehenden Ansprüche zur Verwendung bei der Behandlung von Entzündungsschmerzen, **dadurch gekennzeichnet, dass** es wenigstens ein Polysaccharid oder ein Gemisch aus mehreren Polysacchariden umfasst.

5. Produkt nach einem der vorangehenden Ansprüche zur Verwendung bei der Behandlung von Entzündungsschmerzen, **dadurch gekennzeichnet, dass** es wenigstens ein Polysaccharid umfasst, das 2-Hydroxyethylstärke ist.

6. Produkt nach einem der vorangehenden Ansprüche zur Verwendung bei der Behandlung von Entzündungsschmerzen, **dadurch gekennzeichnet, dass** es wenigstens eine grenzflächenaktive Substanz oder ein Gemisch mehrerer grenzflächenaktiver Substanzen, ausgewählt aus kationischen, anionischen oder nicht-ionischen grenzflächenaktiven Substanzen, umfasst.

7. Produkt nach einem der vorangehenden Ansprüche zur Verwendung bei der Behandlung von Entzündungsschmerzen, **dadurch gekennzeichnet, dass** es wenigstens eine grenzflächenaktive Substanz, ausgewählt aus nicht-ionischen grenzflächenaktiven Substanzen der Gruppe der Polysorbate, umfasst.

8. Produkt nach einem der vorangehenden Ansprüche zur Verwendung bei der Behandlung von Entzündungsschmerzen, **dadurch gekennzeichnet, dass** das Botulinum-Toxin ein Proteinkomplex ist.

9. Produkt nach einem der vorangehenden Ansprüche zur Verwendung bei der Behandlung von Entzündungsschmerzen, **dadurch gekennzeichnet, dass** das Botulinum-Toxin ein Protein ist, das frei von jeglichem Komplexprotein ist.

10. Produkt nach einem der vorangehenden Ansprüche zur Verwendung bei der Behandlung von Entzündungsschmerzen, **dadurch gekennzeichnet, dass** das Botulinum-Toxin ein rekombinantes Protein ist.

11. Produkt nach einem der vorangehenden Ansprüche zur Verwendung bei der Behandlung von Entzündungsschmerzen, **dadurch gekennzeichnet, dass** das Botulinum-Toxin durch intramuskuläre oder subkutane Injektion verabreicht wird.

12. Produkt nach einem der vorangehenden Ansprüche zur Verwendung bei der Behandlung von Entzündungsschmerzen, **dadurch gekennzeichnet, dass** das Opiatderivat oder sein Salz auf einem Weg, ausgewählt aus dem topischen, oralen, parenteralen, intramuskulären, intravenösen oder subkutanen Weg, verabreicht wird.

13. Produkt nach einem der vorangehenden Ansprüche zur Verwendung bei der Behandlung von Entzündungsschmerzen, **dadurch gekennzeichnet, dass** es sich in flüssiger Form präsentiert.
